# EUROPEAN PATENT SPECIFICATION

(11) **EP 1 305 348 B1**
(45) Date of publication and mention of the grant of the patent: **22.09.2004**
(21) Application number: 00950646.0
(22) Date of filing: 26.07.2000
(51) Int. Cl.: C08F 24/00, C07D 493/00

(54) **POLYMERIZATION OF OLEFINIC ORTHOESTERS**
POLYMERISATION VON OLEFINISCHEN ORTHOESTERN
POLYMERISATION D'ORTHOESTERS OLEFINIQUES

(43) Date of publication of application: 02.05.2003
(73) Proprietor: E.I. DU PONT DE NEMOURS AND COMPANY, Wilmington, Delaware 19898 (US)
(72) Inventor: WANG, Lin, Hockessin, DE 19707 (US); DRYSDALE, Neville, Everton, Newark, DE 19702 (US)
(74) Representative: Towler, Philip Dean
(86) International application number: PCT/US2000/020193
(87) International publication number: WO 2002/010230

(56) References cited:
- EP-A- 0 211 598
- WO-A-98/37110
- ISHIZONE T ET AL: "PROTECTION AND POLYMERIZATION OF FUNCTIONAL MONOMERS. 25. SYNTHESIS OF WELL-DEFINED POLYSTYRENE BEARING A TRIOL FUNCTIONALITYBY MEANS OF ANIONIC LIVING POLYMERIZATION OF 4-U(4-(4-VINYLPHENYL)BUTOXY)METHYL-1 -METHYL-2,6,7-TRIOXABICYCLO U2.2.2OCTANE" MACROMOLECULES,US,AMERICAN CHEMICAL SOCIETY. EASTON, vol. 28, no. 14, 3 July 1995 (1995-07-03), pages 4829-4836, XP000532816 ISSN: 0024-9297
- PALMER, CHRISTOPHER J. ET AL: "Novel selective catalytic reduction with tritium: synthesis of the GABAA receptor radioligand 1-(4-ethynylphenyl)-4-[2,3-3H2]propyl- 2,6,7-trioxabicyclo[2.2.2]octane" J. LABELLED COMPD. RADIOPHARM. (1991), 29(7), 829-39 , XP000992368
- HAWKINSON, J. E. ET AL: "Photoaffinity ligands for the [3H]TBOB binding site of the GABAA receptor" J. RECEPT. RES. (1991), 11(1-4), 391-405 , XP000992378

## Description

Disclosed herein is a method for (co)polymerizing selected olefinic orthoesters using certain late transition metal containing catalysts. The (co)polymers produced, which may be hydrolyzed to hydroxyl containing (co)polymers, and the olefinic orthoester monomers, are novel compositions.

### TECHNICAL BACKGROUND

Polyolefins containing functional groups can be made by a variety of methods, and are important items of commerce, being used for example for packaging films and molding resins. Such polymers can be made by free radical and transition metal compound catalyzed processes. Late transition metal catalysts are especially useful for including functional groups in olefin polymers, see for example US5880241, WO98/37110 and WO99/05189. However, none of these references describes the polymerization of olefinic orthoesters. Orthoesters are particularly attractive functional groups because they may be readily hydrolyzed to hydroxyl groups. The olefinic bond is on the "alcoholic part" of the original olefinic orthoester, and hydroxyl groups may be readily reacted to crosslink or otherwise modify the hydroxyl containing polyolefin. This makes such polymers useful for example in coatings, and as curable elastomers.

US5116862 and C. J. Palmer, et al., Journal of Labelled Compounds and Pharmaceuticals, vol. 29, p. 829-839 (1991) disclose certain olefinic orthoesters. None of the orthoesters claimed herein is specifically disclosed in either of these references.

### SUMMARY OF THE INVENTION

This invention concerns a polymerization process, comprising the step of contacting, under polymerizing conditions, an olefin of the formula 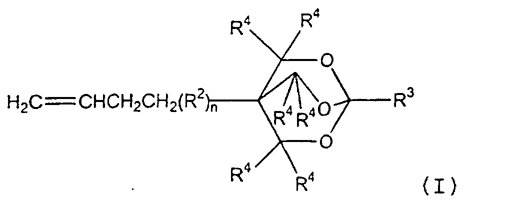 and a nickel or palladium complex of a compound of the formula 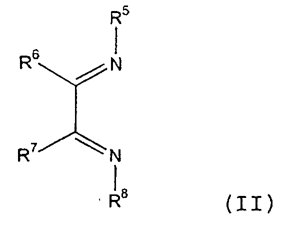 wherein
R² is alkylene or ether substituted alkylene;
n is 0 or 1;
R³ is hydrogen, hydrocarbyl or substituted hydrocarbyl;
each R⁴ is independently hydrogen, a functional group, or a hydrocarbyl or substituted hydrocarbyl not containing an olefinic or acetylenic group;
R⁵ and R⁸ are each independently hydrocarbyl or substituted hydrocarbyl, provided that the atom bound to the imino nitrogen atom has at least two carbon atoms bound to it; and
R⁶ and R⁷ are each independently hydrogen, hydrocarbyl or substituted hydrocarbyl, provided that R⁶ and R⁷ may be taken together to form a carbocyclic ring.

Optionally, one or more olefins of the formula H₂C=CHR¹, wherein R¹ is hydrogen or alkyl, may also be contacted.

This invention also concerns a compound of the formula 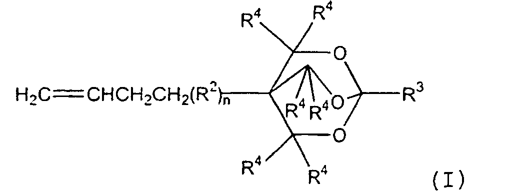 wherein:
R¹ is hydrogen or alkyl;
R² is saturated hydrocarbylene or ether substituted saturated hydrocarbylene;
n is 0 or 1;
R³ is hydrogen, hydrocarbyl or substituted hydrocarbyl; and
each R⁴ is independently hydrogen, a functional group, or a hydrocarbyl or a substituted hydrocarbyl not containing an olefinic or acetylenic group.

Also disclosed herein is a polyolefin having a side chain of the formula 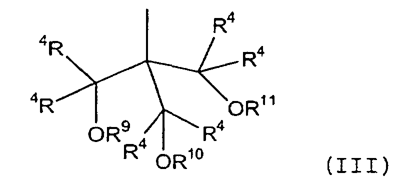 wherein:
each R⁴ is independently hydrogen, a functional group, or a hydrocarbyl or substituted hydrocarbyl not containing an olefinic or acetylenic group;
R⁹, R¹⁰ and R¹¹ are each independently hydrogen or acyl, provided that R⁹, R¹⁰ and R¹¹ taken together may be 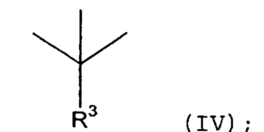 and
R³ is hydrogen, hydrocarbyl or substituted hydrocarbyl.

### DETAILED DESCRIPTION OF THE PREFERRED EMBODIMENTS

Herein, certain terms are used. Some of them are:

A "hydrocarbyl group" is a univalent group containing only carbon and hydrogen. As examples of hydrocarbyls may be mentioned unsubstituted alkyls, cycloalkyls and aryls. If not otherwise stated, it is preferred that hydrocarbyl groups (and alkyl groups) herein contain 1 to about 30 carbon atoms.

By "substituted hydrocarbyl" herein is meant a hydrocarbyl group that contains one or more substituent groups which are inert under the process conditions to which the compound containing these groups is subjected (e.g., an inert functional group, see below). The substituent groups also do not substantially detrimentally interfere with the oligomerization process or operation of the oligomerization catalyst system. If not otherwise stated, it is preferred that substituted hydrocarbyl groups herein contain 1 to about 30 carbon atoms. Included in the meaning of "substituted" are rings containing one or more heteroatoms, such as nitrogen, oxygen and/or sulfur. In a substituted hydrocarbyl, all of the hydrogens may be substituted, as in trifluoromethyl.

By "(inert) functional group" herein is meant a group, other than hydrocarbyl or substituted hydrocarbyl, which is inert under the process conditions to which the compound containing the group is subjected. The functional groups also do not substantially deleteriously interfere with any process described herein that the compound in which they are present may take part in. Examples of functional groups include halo (fluoro, chloro, bromo and iodo), and ether such as -OR²⁴ wherein R²⁴ is hydrocarbyl or substituted hydrocarbyl. In cases in which the functional group may be near a transition metal atom, the functional group alone should not coordinate to the metal atom more strongly than the groups in those compounds that are shown as coordinating to the metal atom, that is they should not displace the desired coordinating group.

By a "cocatalyst" or a "catalyst activator" is meant one or more compounds that react with a transition metal compound to form an activated catalyst species. One such catalyst activator is an "alkyl aluminum compound" which, herein, is meant a compound in which at least one alkyl group is bound to an aluminum atom. Other groups such as, for example, alkoxide, hydride and halogen may also be bound to aluminum atoms in the compound.

By "not containing an olefinic or acetylenic group" is meant the compound does not contain a carbon-carbon triple bond or a non-aromatic carbon-carbon double bond.

By "saturated hydrocarbylene" herein is meant a divalent group containing only carbon and hydrogen, and which contains no carbon-carbon multiple (double, triple or aromatic-type) bonds. Typical saturated hydrocarbylene groups are -(CH₂)₄-, and -CH₂CH(CH₂CH₃)CH₂CH₂-. If not otherwise stated, it is preferred that saturated hydrocarbylene groups herein contain 1 to about 30 carbon atoms.

By "aryl" is meant a monovalent aromatic group in which the free valence is to the carbon atom of an aromatic ring. An aryl may have one or more aromatic rings which may be fused, connected by single bonds or other groups.

By "substituted aryl" is meant a monovalent aromatic group substituted as set forth in the above definition of "substituted hydrocarbyl". Similar to an aryl, a substituted aryl may have one or more aromatic rings which may be fused, connected by single bonds or other groups; however, when the substituted aryl has a heteroaromatic ring, the free valence in the substituted aryl group can be to a heteroatom (such as nitrogen) of the heteroaromatic ring instead of a carbon.

By "neutral Lewis base" is meant a compound, which is not an ion, that can act as a Lewis base. Examples of such compounds include ethers, amines, sulfides and organic nitriles.

By "cationic Lewis acid" is meant a cation that can act as a Lewis acid. Examples of such cations are sodium and silver cations.

By "relatively noncoordinating" (or "weakly coordinating") anions are meant those anions as are generally referred to in the art in this manner, and the coordinating ability of such anions is known and has been discussed in the literature, see for instance W. Beck., et al., Chem. Rev., vol. 88 p. 1405-1421 (1988), and S. H. Stares, Chem. Rev., vol. 93, p. 927-942 (1993). Among such anions are those formed from the aluminum compounds and for example Q⁻ or S⁻ (see below) , including R²³₃AlS⁻, R²³₂AlClQ⁻, R²³AlCl₂Q⁻, and "R²³AlOQ⁻", wherein R²³ is alkyl. Other useful noncoordinating anions include BAF⁻ {BAF = tetrakis[3,5-bis(trifluoromethyl)phenyl]borate}, SbF₆⁻, PF₆⁻, and BF₄⁻, trifluoromethanesulfonate, p-toluenesulfonate, (R_{f}SO₂)₂N⁻, and (C₆F₅)₄B⁻.

The transition metal complexes used in the process disclosed herein contain α-diimine ligands. Such transition metal complexes are known to polymerize various olefins (but not known to polymerize orthoesters) under various conditions. The structures of such complexes, how to prepare them, how to prepare active polymerization catalysts from them (if they are not already active polymerization catalysts), and polymerization conditions are known in the art, see for example US5880241, US5866663, US5852145, WO99/05189, WO97/48739, WO98/49208, WO98/37710, WO98/03167, WO97/48737 and WO97/38024. These references, especially US5880241, describe preferred structures for the α-diimines and their complexes, and these are also preferred herein. In such complexes herein it is preferred that the transition metal is nickel. The α-diimine complex may be added directly to the polymerization as a compound which will directly catalyze polymerization (see Example 3), may be added a complex that is chemically modified (e.g., with a cocatalyst) to form an active polymerization catalyst (see example 4), or ingredients to form the complex in situ may added to the process.

The above references on α-diimine complexes also contain information on various types of polymerization processes (gas phase, solution, slurry, continuous, batch and semi-batch), and conditions and preferred conditions for running such processes. When applicable (for example it is unlikely that gas phase polymerization will result in much incorporation of most orthoester monomers since these monomers are not very volatile), such conditions and preferred conditions are applicable herein.

For instance one method of running the polymerization is to use a complex of the formula 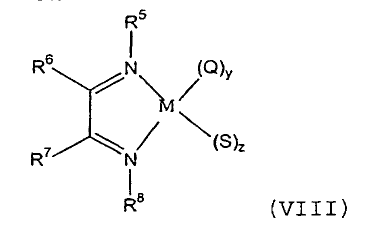 wherein R⁵, R⁶, R⁷ and R⁸ are as defined above, M is Ni or Pd, Q and S are each anions, and y and z are integers so that y+z=m, wherein m is the oxidation state of M. Preferably m is 2. This complex is typically contacted with the olefinic orthoester(s), optionally with one or more other olefinic monomer(s), at a temperature of about -100°C to about +200°C, preferably 0°C to 100°C, and also with a first compound W, which is a neutral Lewis acid capable of abstracting either Q⁻ or S⁻ from (VIII) to form WQ⁻ or WS⁻, provided that the anion formed is a weakly coordinating anion; or a cationic Lewis or Bronsted acid whose counterion is a weakly coordinating anion, to form an active polymerization catalyst. If none of Q⁻ or S⁻ are alkyl, hydride or acyl, either W or a second compound is added that is capable of reacting with (VIII) to remove a Q⁻ or S⁻ and replace it with a hydride, alkyl or acyl anion. Typically W is chosen to be able to do this also, if needed. A typical and preferred W is an alkylaluminum compound, which is not only capable of abstract Q⁻ or S⁻, but will also provide an alkyl group if needed. Useful alkylaluminum compounds include aluminoxanes such as methyl aluminoxane, and alkyl aluminum chlorides such as diethylaluminum chloride, ethylaluminum dichloride and ethylaluminum sesquichloride. Q⁻ and S⁻ may different of the same. Preferred Q and S are halide, especially chloride and bromide, and carboxylate, and chloride and bromide are especially preferred. See Example 4 for such a polymerization.

Any nickel or palladium complex of a bidentate ligand which is capable of (co)polymerizing olefins containing polar groups such as esters, may also be used in the copolymerization herein. Such complexes may be found in US5866663, US5714556, WO98/30610, WO98/30609, WO98/47934 and WO99/05189, WO00/06620 and U.S. Patent Application 09/539560 (filed 31 March 2000).

It is preferred in all processes another olefin is present, and that R¹ is hydrogen or n-alkyl containing 1 to 18 carbon atoms, especially preferably R¹ is hydrogen or methyl, and more preferably R¹ is hydrogen (the olefin is ethylene).

Compounds of the formula (I) can be prepared by the methods as set forth in the Examples (see Examples 1 and 2). Variations in the starting components (and process conditions required to utilize those starting components) will be recognized, or can be determined without undue experimentation, by those of ordinary skill in the art as required to achieve the desired end compound.

In (I) it is preferred that:
n is 1; and/or
R² is alkylene, more preferably that R² is -(CH₂)ₘ- wherein m is 1 to 18; and/or
each R⁴ is hydrogen; and/or
R³ is hydrocarbyl, more preferably alkyl, and especially preferably methyl.

In (II) it is preferred that:
R⁶ and R⁷ are hydrogen, methyl, or taken together are 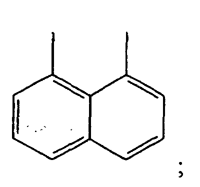 and/or
R⁵ is 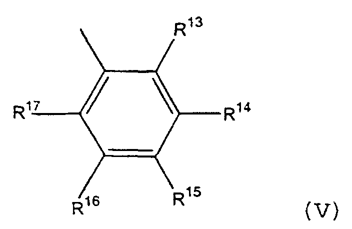
and R⁸ is 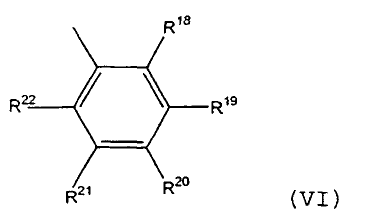
wherein
R¹³ and R¹⁸ are each independently hydrocarbyl, substituted hydrocarbyl or an inert functional group,
R¹⁴, R¹⁵, R¹⁶, R¹⁹, R²⁰ and R²¹ are each independently hydrogen, hydrocarbyl, substituted hydrocarbyl or an inert functional group, and
R¹⁷ and R²² are each independently hydrogen, hydrocarbyl, substituted hydrocarbyl or an inert functional group, and provided that any two of R¹³, R¹⁴, R¹⁵, R¹⁶, R¹⁷, R¹⁸, R¹⁹, R²⁰, R²¹ and R²² that are vicinal to one another, taken together may form a ring.

In (III) it is preferred that:
R⁹, R¹⁰ and R¹¹ are all hydrogen; or
R⁹ and R¹⁰ are hydrogen and R¹¹ is acyl; and/or
acyl is R²⁵C(O)- wherein R²⁵ is hydrocarbyl or substituted hydrocarbyl, more preferably hydrocarbyl, especially preferably alkyl, and very preferably methyl.

(III) is a pendant group, and in (III) the bond not labeled is a free bond. By a pendant group is meant that (III) is connected to the main polymer chain either directly by the free bond, or being connected through an intermediate divalent groups such as -CH₂CH₂R²-. However since "chain walking" may take place during some of these polymerizations (see previously incorporated US5880241), these connecting groups may vary.

In structure (IV) the 3 bonds which have no groups at the ends are free covalent bonds, not methyl groups.

Polyolefins in which (III) is present in the ester or orthoester form may be hydrolyzed by standard methods for hydrolyzing these functional groups, such as reaction with water and/or alcohols. These hydrolyses are usually acid and/or base catalyzed reactions.

Polyolefins which contain (III) in which at least one of R⁹, R¹⁰, or R¹¹ is hydrogen may be crosslinked by any reaction known to crosslink hydroxyl containing polymers may be used. For instance, the polymer may be reacted with di-or higher functional epoxides, isocyanates, carboxylic acids, acyl halides, esters, carboxylic anhydrides and organic carbonates. These crosslinking compounds may be monomeric (of low molecular weight) or polymeric. If the polyolefin containing (III) happens to be elastomeric, a crosslinked elastomer results. These elastomers may be particularly useful as sealants and caulks. Various methods are known in the art for delaying such crosslinking reactions, such as masking the one of the groups taking place in the crosslinking reaction and "activating" by heating.

In the Examples, the following abbreviations are used:
DSC - Differential Scanning Calorimetry
PMAO-IP - poly(methylaluminoxane) (Akzo-Nobel Inc.)
RB - round-bottomed
RT - room temperature
TCE - 1,1,2,2-tetrachloroethane
THF - tetrahydrofuran
Melting points of polymers are taken as the peak of the melting endotherm.

### Example 1

### Preparation of 2,2-Bis(hydroxymethyl)-10-undecen-1-ol

In a 300 mL RB flask equipped with a stirrer and reflux condenser were added undecylenic aldehyde (10-undecenal) (20.98 g) and 37% formaldehyde (83.24 g). To this solution a 50% solution of sodium hydroxide (10.41 g) was added at such a rate so that the temperature remained below 50°C. After the addition, the solution was heated at 90°C for one h. The solution was allowed to cooled to RT and then neutralized with formic acid. The solution was then concentrated at reduced pressure, then chloroform (250 mL) added. The resulting mixture was filtered through Celite® and the filtrate concentrated at reduced pressure and then vacuum distilled. The fraction boiling at 142-151°C at -200 Pa (absolute) was collected (13.99 g, 48.66% yield).

### Example 2

### Preparation of 1-Methyl-4-(8-nonenyl)-2,6,7-trioxabicyclo[2.2.2]octane (VII)

In a oven dried 250 mL RB flask equipped with a stirrer, thermometer and a distillation head were add 2,2-bis-(hydroxymethyl)-10-undecen-1-ol (38.0 g), triethyl orthoacetate (28.4 g), dioctyl phthalate (132.2 g) and 4-ethylbenzene sulfonic acid (0.132 g). The resulting mixture was placed under nitrogen and heated to 140°C during which the theoretical amount of ethanol was distilled off. The reaction was allowed to cool and then placed under vacuum. When a vacuum of less than 133 Pa was achieved the reaction was heated to a maximum temperature of 220°C during which the desired product distilled off as a clear liquid (39.05 g, 93.06% yield). This was then redistilled, ¹H NMR (CDCl₃) : 5.75 (m, 1H), 4.9 (m, 2H), 3.85 (s, 6H), 2.00 (m, 2H), 1.30 (s, 3H), 1.4-1.0 (m, 12H).

### Example 3

### Synthesis of an Ethylene Copolymer of CH₂=CH(CH₂)₇C(CH₂O)₃CCH₃ Using a Pd Catalyst

In a drybox, (IX) (0.0848 g, 0.1 mmol), and CH₂=CH(CH₂)₇C(CH₂O)₃CCH₃ [(VII), 5.0 g, 0.0294 mole] were dissolved in 40 mL CH₂Cl₂ in a 200 mL Schlenk flask. This was brought out of the drybox and was purged with 1 atm ethylene for 15 min. The solution was then stirred under 1 atm of ethylene for 48 hr at RT. The mixture was then poured into a 500 mL beaker and was added 300 mL methanol. The polymer was isolated and was redissolved in 50 mL CH₂Cl₂. To this was added 300 mL methanol. The isolated polymer was dried overnight in vacuo at 50°C. A viscous oil (13.5 g) was obtained. Based on ¹H NMR(CD₂Cl₂), the orthoester incorporation was 2.8 mole % {δ 3.88 (s, 6H, -C*H*₂-O-); -C*Me*- resonances overlapped with that of the methyls (0.8-1.0) on the copolymers, the methylene peaks overlapped with that of the copolymers (1.1-1.4). The polymer was branched based on ¹H NMR (91 Me/1000CH₂). Gel permeation chromatography (THF, polystyrene standard): Mw = 186,485; Mn = 61,384; Mw/Mn = 3.0.

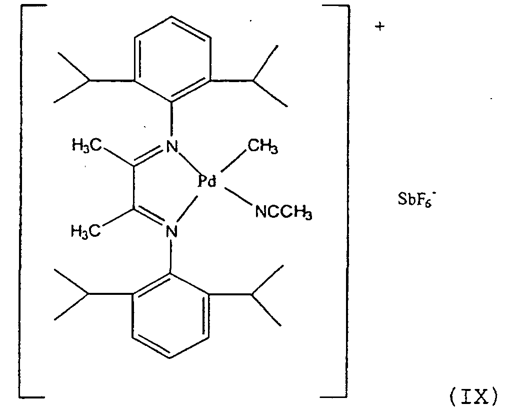

### Example 4

### Synthesis of an Ethylene Copolymer of CH₂=CH(CH₂)₇C(CH₂O)₃CCH₃ Using a Ni catalyst

In a drybox, (X) (8.7 mg, 0.017 mmol), CH₂=CH(CH₂)₇C(CH₂O)₃CCH₃ [(VII), 5.0 g], and 35 mL toluene were mixed in a 200 mL Schlenk flask. The mixture was brought out of the drybox and was placed under 1 atm of ethylene. The mixture was purged with ethylene while cooled in an ice-water bath for 15 min. PMAO-IP (1.2 mL, 13.5 wt% toluene solution) was injected into the reaction mixture and this was allowed to stir for 3 h at 0°C under 1 atm of ethylene. To the mixture was slowly added 100 mL methanol. The mixture was allowed to stir at RT for 30 min. White solid copolymer was filtered, washed with 6X20 mL methanol and dried in full vacuo at 100°C overnight. White polymer (6.15 g) was obtained. Both ¹H NMR and ¹³C NMR showed that comonomer was incorporated, but was likely in the alcohol form since there were no orthoester or ester groups in the polymer. ¹H NMR(TCE-*d*_{*2*}, 120°C): δ 3.52 (d, 6H, -C*H*_{*2*}-OH), 1.2-1.4 (s, -C*H*_{*2*}- of the polymer), 0.8-1.0 (m, -C*H*_{*3*} of the polymer). The comonomer incorporation was 0.6 mole %. The polymer had 5Me/1000CH₂. DSC(10°C/min, 2^{nd} heat) : m.p. = 121°C (94.6 J/g).

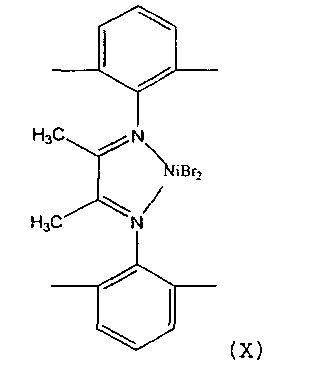

### Example 5

### Hydrolysis of Polymer

The poly(olefin-orthoester) made in Example 3 (1.0 g) was dissolved in THF (10 mL) then 0.20 mL of a 0.242 M solution of p-toluenesulfonic acid added. Aliquots were withdrawn after 2 and 4 h and the solvent removed from each aliquot under vacuum. ¹H NMR showed the complete hydrolysis of the orthoester after 2 h. After stirring overnight, the THF was removed from the main portion of the reaction under vacuum and chloroform added. The resulting solution was washed with 5% sodium carbonate (2 x 10 mL) and then with water. The organic phase was dried over sodium sulfate, filtered, concentrated and then dried under vacuum affording 0.55 g of the polyol. IR showed two carbonyl resonances at 1744 and 1722 cm⁻¹, indicating partial hydrolysis of the orthoester and/or carboxylic ester groups (which result from hydrolysis of the orthoester).

### Comparative Example A

In a drybox, (IX) (0.0848 g, 0.1 mmol), and CH₂=CHCH₂C(CH₂O)₃CCH₃ (5.0 g, 0.0294 mole), were dissolved in 40 mL CH₂Cl₂ in a 200 mL Schlenk flask. This was brought out of the drybox and was purged with 1 atm ethylene for 15 min. The solution was then stirred under 1 atm of ethylene for 48 hr at RT. The mixture was then poured into a 500 mL beaker and 300 mL methanol was added. The polymer was isolated and was redissolved in 50 mL CH₂Cl₂. To this was added 300 mL methanol. The isolated polymer was dried overnight in vacuo at 50°C. Viscous oil (22 g) was obtained. Based on ¹H NMR(CD₂Cl₂), no orthoester monomer was incorporated. The polymer was polyethylene. Gel permeation chromatography (THF, polystyrene standard): Mw = 59,536; Mn = 33,872; Mw/Mn = 1.8.

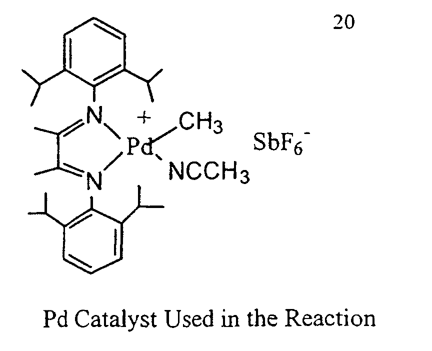

## Claims

1. A polymerization process comprising the step of contacting, under polymerizing conditions, one or more olefins and a nickel or palladium complex of a compound of the formula 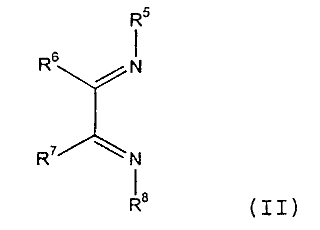 wherein
R⁵ and R⁸ are each independently hydrocarbyl or substituted hydrocarbyl, provided that the atom bound to the imino nitrogen atom has at least two carbon atoms bound to it; and
R⁶ and R⁷ are each independently hydrogen, hydrocarbyl or substituted hydrocarbyl, provided that R⁶ and R⁷ may be taken together to form a carbocyclic ring,
**characterized in that** an olefin is of the formula 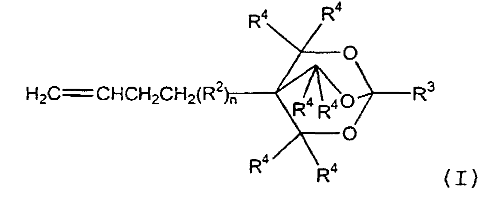 wherein
R² is alkylene or ether substituted alkylene;
n is 0 or 1;
R³ is hydrogen, hydrocarbyl or substituted hydrocarbyl;
each R⁴ is independently hydrogen, a functional group, or a hydrocarbyl or substituted hydrocarbyl not containing an olefinic or acetylenic group.

2. The process as recited in claim 1, **characterized in that** said complex is 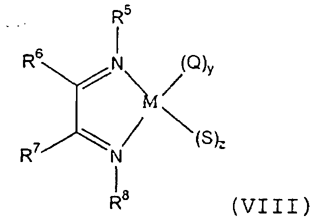 wherein:
M is Ni or Pd;
Q and S are each independently anions; and
y and z are integers so that y+z=m, wherein m is the oxidation state of M;
and wherein said complex is contacted with a first compound W, which is a neutral Lewis acid capable of abstracting either Q⁻ or S⁻ to form WQ⁻ or WS⁻, provided that the anion formed is a weakly coordinating anion; or a cationic Lewis or Bronsted acid whose counterion is a weakly coordinating anion, to form an active polymerization catalyst, provided that if none of Q⁻ or S⁻ are alkyl, hydride or acyl, either W or a second compound is added which is capable of reacting with said complex to remove a Q⁻ or S⁻ and replace it with a hydride, alkyl or acyl anion.

3. The process as recited in claim 2, **characterized in that** W is an alkylaluminum compound.

4. The process as recited in claim 1, **characterized in that** the complex and the olefin are contacted at a temperature of 0°C to 100°C.

5. The process as recited in claim 1, **characterized in that**:
n is 1;
R² is -(CH₂)ₘ- wherein m is 1 to 18; and/or
each R⁴ is hydrogen;
R³ is hydrocarbyl;
R⁶ and R⁷ are hydrogen, methyl, or taken together are 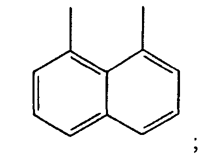
R⁵ is 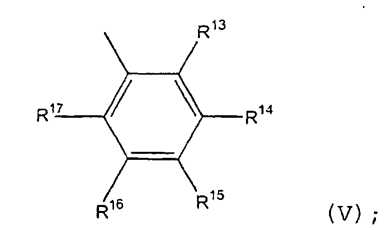 and
R⁸ is 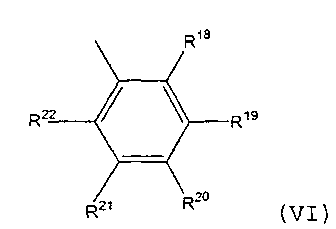 wherein:
R¹³ and R¹⁸ are each independently hydrocarbyl, substituted hydrocarbyl or an inert functional group;
R¹⁴, R¹⁵, R¹⁶, R¹⁹, R²⁰ and R²¹ are each independently hydrogen, hydrocarbyl, substituted hydrocarbyl or an inert functional group; and
R¹⁷ and R²² are each independently hydrogen, hydrocarbyl, substituted hydrocarbyl or an inert functional group;
and provided that any two of R¹³, R¹⁴, R¹⁵, R¹⁶, R¹⁷, R¹⁸, R¹⁹, R²⁰, R²¹ and R²² that are vicinal to one another, taken together may form a ring.

6. The process as recited in any one of claims 1-5, **characterized in that** at least one olefin of the formula H₂C=CHR¹ is present, wherein R¹ is hydrogen or alkyl.

7. The process as recited in claim 6, **characterized in that** R¹ is hydrogen.

8. A compound of the formula 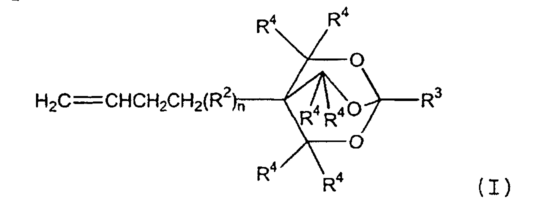 wherein:
R² is saturated hydrocarbylene or ether substituted saturated hydrocarbylene;
n is 0 or 1;
R³ is hydrogen, hydrocarbyl or substituted hydrocarbyl; and
each R⁴ is independently hydrogen a functional group, or hydrocarbyl or substituted hydrocarbyl not containing an olefinic or acetylenic group.

9. The compound as recited in claim 8, **characterized in that** n is 1; R² is alkylene; each R⁴ is hydrogen; and R³ is hydrocarbyl.

10. The compound as recited in claim 9, **characterized in that** R² is -(CH₂)ₘ- wherein m is 1 to 18; and R³ is alkyl.

11. A polyolefin having a side chain of the formula 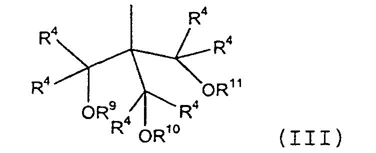 wherein:
each R⁴ is independently hydrogen, a functional group, or a hydrocarbyl or substituted hydrocarbyl not containing an olefinic or acetylenic group;
R⁹, R¹⁰ and R¹¹ are each independently hydrogen or acyl, or R⁹, R¹⁰ and R¹¹ taken together are 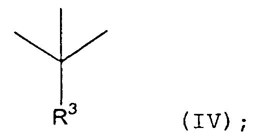 and
R³ is hydrogen, hydrocarbyl or substituted hydrocarbyl.

12. The polyolefin as recited in claim 12, **characterized in that** each R⁴ is hydrogen.

13. The polyolefin as recited in claim 11 or 12, **characterized in that** each R³ is hydrogen.

14. The polyolefin as recited in claim 11 or 12 which has been crosslinked.

## Patentansprüche

1. Verfahren zur Polymerisation, umfassend den Schritt des Kontaktierens unter polymerisierenden Bedingungen von einem oder mehreren Olefinen und eines Nickeloder Palladium-Komplexes einer Verbindung der Formel: 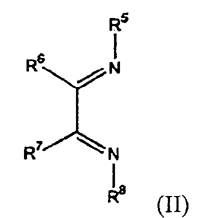 worin sind:
R⁵ und R⁸ jeweils unabhängig Hydrocarbyl oder substituiertes Hydrocarbyl unter der Voraussetzung, dass das an dem Imino-Stickstoffatom gebundene Atom mindestens über 2 Kohlenstoffatome verfügt, die an diesem gebunden sind; und
R⁶ und R⁷ jeweils unabhängig Wasserstoff, Hydrocarbyl oder substituiertes Hydrocarbyl unter der Voraussetzung, dass R⁶ und R⁷ unter Bildung eines carbocyclischen Ringes zusammengenommen werden können;
**dadurch gekennzeichnet, dass** ein Olefin die Formel hat: 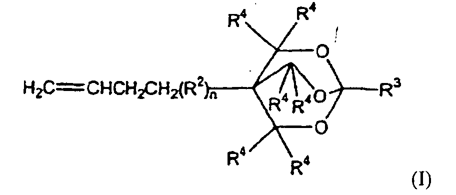 worin sind:
R² Alkylen oder Ether-substituiertes Alkylen;
n Null oder 1;
R³ Wasserstoff, Hydrocarbyl oder substituiertes Hydrocarbyl; und
jedes R⁴ unabhängig Wasserstoff, eine funktionelle Gruppe, ein Hydrocarbyl oder substituiertes Hydrocarbyl, das keine olefinische oder acetylenische Gruppe enthält.

2. Verfahren nach Anspruch 1, **dadurch gekennzeichnet, dass** der Komplex 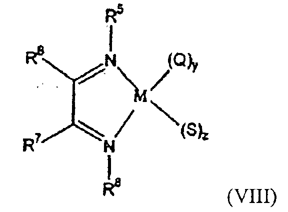 ist und worin sind:
M Ni oder Pd;
Q und S jeweils unabhängig Anionen und
y und z ganze Zahlen, so dass y+z=m gilt, worin m die Oxidationsstufe von M
ist;
und worin der Komplex kontaktiert wird mit einer ersten Verbindung W, die eine neutrale Lewis-Säure ist, die entweder Q⁻ oder S⁻ abspalten kann, um WQ⁻ oder WS⁻ zu bilden unter der Voraussetzung, dass das erzeugte Anion ein schwach koordinierendes Anion ist; oder eine kationische Lewis- oder Brönsted-Säure, deren Gegen-Ion ein schwach koordinierendes Anion ist, um einen aktiven Polymerisationskatalysator zu erzeugen unter der Voraussetzung, dass, wenn kein Q⁻ oder S⁻ Alkyl, Hydrid oder Acyl ist, dann entweder W oder eine zweite Verbindung zugesetzt wird, die in der Lage ist, mit dem Komplex unter Entfernung von Q⁻ und S⁻ zu reagieren und diese durch ein Hydrid-, Alkyl- oder Acyl-Anion zu ersetzen.

3. Verfahren nach Anspruch 2, **dadurch gekennzeichnet, dass** W eine Alkylaluminiumverbindung ist.

4. Verfahren nach Anspruch 1, **dadurch gekennzeichnet, dass** der Komplex und das Olefin kontaktiert werden bei einer Temperatur von 0° bis 100°C.

5. Verfahren nach Anspruch 1, **dadurch gekennzeichnet, dass**:
n 1 beträgt;
R²-(CH₂)ₘ- ist, worin m 1 bis 18 beträgt; und/oder
jedes R⁴ Wasserstoff ist;
R³ ist Hydrocarbyl;
R⁶ und R⁷ sind Wasserstoff, Methyl oder sind zusammengenommen 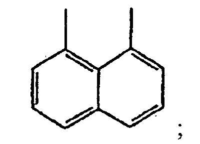
R⁵ ist 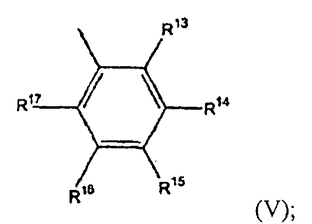
und R⁸ ist 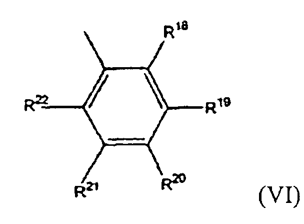
worin sind:
R¹³ und R¹⁸ jeweils unabhängig Hydrocarbyl, substituiertes Hydrocarbyl oder eine inerte funktionelle Gruppe;
R¹⁴, R¹⁵, R¹⁶, R¹⁹, R²⁰ und R²¹ jeweils unabhängig Wasserstoff, Hydrocarbyl, substituiertes Hydrocarbyl oder eine inerte funktionelle Gruppe; und
R¹⁷ und R²² jeweils unabhängig Wasserstoff, Hydrocarbyl, substituiertes Hydrocarbyl oder eine inerte funktionelle Gruppe;
und unter der Voraussetzung, dass jeweils zwei von R¹³, R¹⁴, R¹⁵, R¹⁶, R¹⁷, R¹⁸, R¹⁹, R²⁰, R²¹ und R²², die zueinander nachbarständig sind, zusammengenommen einen Ring bilden können.

6. Verfahren nach einem der Ansprüche 1 bis 5, **dadurch gekennzeichnet, dass** mindestens ein Olefin der Formel H₂C=CHR¹ vorhanden ist, worin R¹ Wasserstoff oder Alkyl ist.

7. Verfahren nach Anspruch 6, **dadurch gekennzeichnet, dass** R¹ Wasserstoff ist.

8. Verbindung der Formel 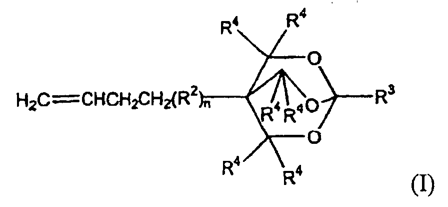 worin sind:
R² gesättigtes Hydrocarbylen oder Ether-substituiertes gesättigtes Hydrocarbylen;
n Null oder 1;
R³ Wasserstoff, Hydrocarbyl oder substituiertes Hydrocarbyl; und
jedes R⁴ unabhängig Wasserstoff, eine funktionelle Gruppe oder Hydrocarbyl oder substituiertes Hydrocarbyl, die keine olefinische oder acetylenische Gruppe enthalten.

9. Verbindung nach Anspruch 8, **dadurch gekennzeichnet, dass** n 1 beträgt; R² ist Alkylen; jedes R⁴ ist Wasserstoff und R³ ist Hydrocarbyl.

10. Verbindung nach Anspruch 9, **dadurch gekennzeichnet, dass** R² -(CH₂)ₘ- ist, worin m 1 bis 18 beträgt und R³ ist Alkyl.

11. Polyolefin mit einer Seitenkette der Formel: 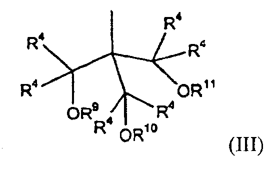 worin sind:
jedes R⁴ unabhängig Wasserstoff, eine funktionelle Gruppe oder Hydrocarbyl oder substituiertes Hydrocarbyl, die keine olefinische oder acetylenische Gruppe enthalten;
R⁹, R¹⁰ und R¹¹ jeweils unabhängig Wasserstoff oder Acyl oder R⁹, R¹⁰ und R¹¹ sind zusammengenommen 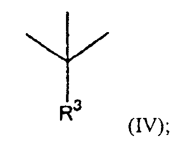 und
R³ Wasserstoff, Hydrocarbyl oder substituiertes Hydrocarbyl.

12. Polyolefin nach Anspruch 11, **dadurch gekennzeichnet, dass** jedes R⁴ Wasserstoff ist.

13. Polyolefin nach Anspruch 11 oder 12, **dadurch gekennzeichnet, dass** jedes R³ Wasserstoff ist.

14. Polyolefin nach Anspruch 11 oder 12, das vernetzt worden ist.

## Revendications

1. Procédé de polymérisation comprenant l'étape de mise en contact, sous des conditions de polymérisation, d'une ou plusieurs oléfine(s) et d'un complexe de nickel ou de palladium d'un composé de la formule 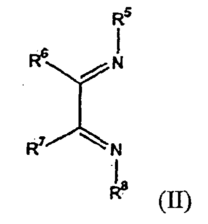 dans laquelle
R⁵ et R⁸ représentent chacun indépendamment un hydrocarbyle ou un hydrocarbyle substitué, à condition que l'atome lié à l'atome d'azote du groupe imino ait au moins deux atomes de carbone qui lui soient liés; et
R⁶ et R⁷ représentent chacun indépendamment l'hydrogène, un hydrocarbyle ou un hydrocarbyle substitué, à condition que R⁶ et R⁷ puissent être pris ensemble pour former un cycle carbocyclique,
**caractérisé en ce qu'**une oléfine est de la formule 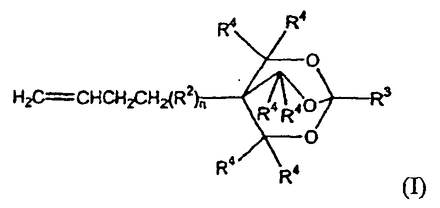 dans laquelle
R² représente un alkylène ou un alkylène substitué par un éther;
n représente 0 ou 1;
R³ représente l'hydrogène, un hydrocarbyle ou un hydrocarbyle substitué;
chaque R⁴ représente indépendamment l'hydrogène, un groupe fonctionnel, ou un hydrocarbyle ou un hydrocarbyle substitué ne contenant pas de groupe oléfinique ni acétylénique.

2. Procédé tel qu'énoncé dans la revendication 1, **caractérisé en ce que** ledit complexe est 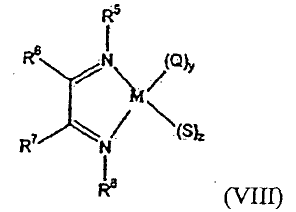 dans laquelle:
M représente Ni ou Pd;
Q et S représentent chacun indépendamment des anions; et
y et z représentent des nombres entiers de sorte que y + z = m, dans lequel m représente l'état d'oxydation de M;
et dans lequel ledit complexe est mis en contact avec un premier composé W, qui est un acide de Lewis neutre capable de soustraire soit Q⁻ soit S⁻ pour former WQ⁻ ou WS⁻, à condition que l'anion formé soit un anion qui se coordonne plus faiblement; ou un acide cationique de Lewis ou de Bronsted dont le contre-ion est un anion qui se coordonne plus faiblement, pour former un catalyseur actif de polymérisation, à condition que si aucun de Q⁻ ou de S⁻ ne représente un alkyle, un hydrure ou un acyle, soit W soit un second composé est ajouté qui capable de réagir avec ledit complexe pour retirer un Q⁻ ou un S⁻ et le remplacer par un anion hydrure, alkyle ou acyle.

3. Procédé tel qu'énoncé dans la revendication 2, **caractérisé en ce que** W représente un composé alkylaluminium.

4. Procédé tel qu'énoncé dans la revendication 1, **caractérisé en ce que** le complexe et l'oléfine sont mis en contact à une température de 0°C à 100°C.

5. Procédé tel qu'énoncé dans la revendication 1, **caractérisé en ce que**
n représente 1;
R² représente -(CH₂)ₘ- dans lequel m représente 1 à 18; et/ou
chaque R⁴ représente l'hydrogène;
R³ représente un hydrocarbyle;
R⁶ et R⁷ représentent l'hydrogène, un méthyle, ou pris ensemble sont 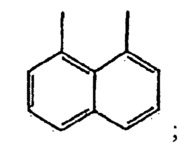
R⁵ représente 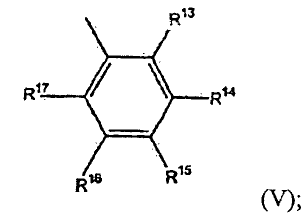 et
R⁸ représente 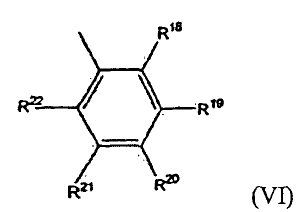
dans lesquelles:
R¹³ et R¹⁸ représentent chacun indépendamment un hydrocarbyle, un hydrocarbyle substitué ou un groupe fonctionnel inerte;
R¹⁴, R¹⁵, R¹⁶, R¹⁹, R²⁰ et R²¹ représentent chacun indépendamment l'hydrogène, un hydrocarbyle, un hydrocarbyle substitué ou un groupe fonctionnel inerte; et
R¹⁷ et R²² représentent chacun indépendamment l'hydrogène, un hydrocarbyle, un hydrocarbyle substitué ou un groupe fonctionnel inerte;
et à condition que n'importe lesquels de deux groupes R¹³, R¹⁴, R¹⁵, R¹⁶, R¹⁷, R¹⁸, R¹⁹, R²⁰, R²¹ et R²² qui sont vicinaux l'un par rapport à l'autre, pris ensemble puissent former un cycle.

6. Procédé tel qu'énoncé dans l'une quelconque des revendications 1 à 5, **caractérisé en ce qu'**au moins une oléfine de la formule H₂C=CHR¹ est présente, dans laquelle R¹ est l'hydrogène ou un alkyle.

7. Procédé tel qu'énoncé dans la revendication 6, **caractérisé en ce que** R¹ représente l'hydrogène.

8. Composé de la formule 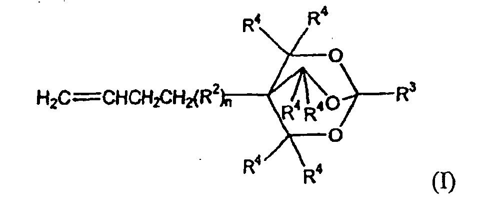 dans laquelle:
R² représente un hydrocarbylène saturé ou un hydrocarbylène saturé substitué par un éther;
n représente 0 ou 1;
R³ représente l'hydrogène, un hydrocarbyle ou un hydrocarbyle substitué; et
chaque R⁴ représente indépendamment l'hydrogène, un groupe fonctionnel, ou un hydrocarbyle ou un hydrocarbyle substitué ne contenant pas de groupe oléfinique ni acétylénique.

9. Composé tel qu'énoncé dans la revendication 8, **caractérisé en ce que** n représente 1; R² représente un alkylène; chaque R⁴ représente l'hydrogène; et R³ représente un hydrocarbyle.

10. Composé tel qu'énoncé dans la revendication 9, **caractérisé en ce que** R² représente -(CH₂)ₘ- dans lequel m représente 1 à 18; et R³ représente un alkyle.

11. Polyoléfine ayant une chaîne latérale de la formule 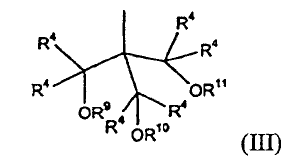 dans laquelle:
chaque R⁴ représente indépendamment l'hydrogène, un groupe fonctionnel, ou un hydrocarbyle ou un hydrocarbyle substitué ne contenant pas de groupe oléfinique ni acétylénique;
R⁹, R¹⁰ et R¹¹ représentent chacun indépendamment l'hydrogène ou un acyle, ou R⁹, R¹⁰ et R¹¹ pris ensemble sont 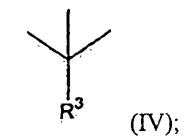 et
R³ représente l'hydrogène, un hydrocarbyle ou un hydrocarbyle substitué.

12. Polyoléfine tel qu'énoncée dans la revendication 11, **caractérisée en ce que** chaque R⁴ représente l'hydrogène.

13. Polyoléfine tel qu'énoncée dans la revendication 11 ou 12, **caractérisée en ce que** chaque R³ représente l'hydrogène.

14. Polyoléfine tel qu'énoncée dans la revendication 11 ou 12 qui a été réticulée.
